# EUROPÄISCHE PATENTANMELDUNG

(11) **EP 1 437 120 A1**
(43) Veröffentlichungstag der Anmeldung: **14.07.2004**
(21) Anmeldenummer: 03025946.9
(22) Anmeldetag: 13.11.2003
(51) Int. Cl.: A61K 7/06, A61K 7/42

(54) **Zum Abspülen bestimmtes kosmetisches Mittel mit UV-Schutz**

(30) Priorität: 11.01.2003 DE 10300762
(71) Anmelder: Wella Aktiengesellschaft, 64274 Darmstadt (DE)
(72) Erfinder: Pfaffernoschke, Matthias, Dr., 6208 Oberkirch (CH); Kolly, Maryline, 1762 Givisiez (CH)

(57) **Zusammenfassung**

Zum Ausspülen bzw. Abspülen bestimmtes kosmetisches Mittel mit Schutzwirkung für Haare und/oder Haut vor UV-Strahlen, dadurch gekennzeichnet, dass es eine Kombination aus einem UV-Quat und einer UV-Filtersubstanz enthält.

## Beschreibung

Gegenstand der vorliegenden Erfindung sind zum Aus- oder Abspülen bestimmte kosmetische Mittel mit UV-Schutz, enthaltend eine Kombination aus einem UV-Quat und einer UV-Filtersubstanz.

Es ist seit langem bekannt, dass ultraviolettes Licht eine schädigende Wirkung auf ungeschützte Haut und Haare des Menschen ausübt. Hierfür ist ursächlich derjenige UV-Anteil des Sonnenlichts verantwortlich, der nicht von der Ozonschicht und dem Luftsauerstoff absorbiert wird. Das von der Erdatmosphäre weggefilterte UV-Licht liegt im Bereich einer Wellenlänge von etwa < 290 nm und ist als UVC bekannt. Schädigende Wirkungen auf der Körperoberfläche bewirken daher Strahlungen im Bereich UVA mit einer Wellenlänge zwischen ca. 400 - 320 nm und UVB mit einer Wellenlänge zwischen ca. 320 - 280 nm, da diese die atmosphärischen Gasschichten durchdringen können.

Die biologischen Wirkungen von UVA und UVB sind vielfältig. Neben den erwünschten Reaktionen in der Haut, wie zum Beispiel der Bildung von Vitamin D aus Steroidvorstufen, verursachen UVA und UVB ein breites Spektrum an Schäden an ungeschützten Teilen der Körperoberfläche, wovon Haut und Haare betroffen sind. Diese Schäden umfassen insbesondere einfache bis schwere Sonnenbrände, Erytheme, Hautnekrosen, vorzeitige Alterung, Tumore oder Strukturveränderungen von Haaren. Das Entstehen von malignen Hauttumoren aufgrund wiederholter Expositionen mit Sonnenlicht muss als gesichert betrachtet werden, so dass vorsorgliche Maßnahmen gegen UVA und UVB Strahlen besonders begründet sind. In jüngerer Zeit kommt nicht zuletzt wegen der Abnahme der Ozonkonzentration in der oberen Erdatmosphäre dem Schutz vor UVA und UVB eine immer größere Bedeutung zu.

Aus diesen Gründen müssen hohe Anforderungen an ein wirksames Mittel gestellt werden. Idealerweise soll ein wirksames Mittel einen ausreichend hohen Lichtschutzfaktor (SPF) und eine hohe Wasserfestigkeit aufweisen, es soll möglichst geringe Konzentrationen an UV-Licht absorbierenden Stoffen beinhalten, es soll gegen UVA und UVB gleichermaßen gut schützen, haut- und haarverträglich sein und die einzelnen Komponenten sollen kompatibel sein.

Klassischerweise werden Haut und Haare vor UV-Strahlung durch den Einsatz von UV-Filtern geschützt. Dies funktioniert jedoch nur, wenn und solange die UV-Filter am Haar bzw. der Haut verbleiben, d. h. im wesentlichen nur bei Leave-in-Rezepturen.

Es besteht aus den genannten Gründen ein Bedürfnis, verbesserte und ausspülbare Lichtschutzmittel bereitzustellen, die alle Erfordernisse an ein wirksames, langanhaltendes und haut- und haarfreundliches Mittel in sich vereinigen.

Aufgabe der vorliegenden Erfindung ist daher ein Mittel mit einem Schutz vor UV-Strahlen bereitzustellen, welches verbesserte Eigenschaften gegenüber den bekannten Mitteln aufweist, die Nachteile des Standes der Technik beseitigt und für Rinse-off-Formulierungen, d. h. Mittel, die dazu bestimmt sind, nach der Anwendung und abgespült zu werden, einen UV-Schutz ermöglicht. Typische Rinse-off-Formulierungen sind Mittel wie Shampoos, Rinses oder Haarkurpackungen, die nach dem Auftragen und einer Einwirkungszeit von 1 bis 60 Minuten, vom Haar wieder abgespült werden.

Die Aufgabe wurde erfindungsgemäß durch Bereitstellen eines Mittels gemäß Anspruch 1 gelöst.

Nähere Ausgestaltungen der vorliegenden Erfindungen sind in den weiteren Patentansprüchen dargestellt.

UV-Quats können sich, da sie eine quaternisierte Kopfgruppe haben, die sich analog den "normalen Quats" verhält, am negativ geladenen Haar substantiv anheften. Es wurde jedoch überraschend bei der Messungen der DSC-Peaktemperatur, welche ein Mass für die Haarschädigung, u. a. hervorgerufen durch UV-Strahlung, ist, eine signifikante Verbesserung (Erhöhung der Peak-Temperatur) durch Kombination mindestens eines UV-Quats mit mindestens einem UV-Filter gefunden. Generell gilt, je höher die DSC-Peaktemperatur, desto weniger geschädigt ist das Haar.

Die DSC (Dynamic Scanning Calorimetry), nach DIN 51005: 1983-11, auch als DDK (*Dynam. Differenz-Kalorimetrie*) oder DWDK (*Dynam. Wärmestrom-Differenz-Kalorimetrie*) bezeichnet, ist ein Verfahren der thermischen Analyse, bei der durch den Vergleich zwischen der Temperaturabhängigkeit der Wärmeaufnahme bzw. -abgabe einer Probe und einer Referenzsubstanz charakteristische Daten (bei Polymeren z. B. die Glasübergangtemperatur) bestimmt werden können.

Die DSC-Messung zeigt in Tabelle 1 anschaulich folgendes: Ein Rinse-Conditioner mit UV-Filter weist lediglich eine Peaktemperatur von 138,80°C auf und ein Rinse-Conditioner mit UV-Quat eine Peaktemperatur von 139,83°C; werden aber erfindungsgemäss UV-Filter und UV-Quat kombiniert, so liegt die Peaktemperatur bei 142,95°C und entspricht damit fast der Peaktemperatur des unbestrahlten Haares von 143,75°C.

Hier ist also tatsächlich ein Schutzeffekt nachweisbar, der offensichtlich auf einem Synergismus zwischen UV-Filter und UV-Quat beruht. Dies ließ sich auch durch Reisskraftmessungen an bestrahlten Haaren bestätigen.

Es wurde ferner unerwartet gefunden, dass durch die synergistische Kombination aus UV-Filter und UV-Quat eine wesentlich verbesserte UV-Schutzwirkung am Haar besonders bei kationischen Rinse-off-Produkten erreicht werden kann. Hierbei ist es unerheblich, ob als UV-Filter UV-Licht absorbierende anorganische Pigmente in dem betreffenden Mittel enthalten sind oder ob die darin enthaltenen organischen UV-Filter wasserlöslich oder öllöslich sind. Die Erklärung für diesen überraschenden Effekt liegt offensichtlich darin, dass der UV-Filter nicht oder nur in geringem Umfang abgespült wird, wenn er in Kombination mit einem UV-Quat eingesetzt wird, wobei zur Erzielung dieses Effekts ein Gewichtsunterschuss an UV-Quat gegenüber UV-Filter ausreicht.

Als UV-Quat kommen alle handelsüblichen UV-Quats in Betracht, welche mindestens eine quaternäre Ammoniumgruppe sowie mindestens eine UV-Licht absorbierende Gruppe aufweisen. Die UV-Quats absorbieren in folgendem Bereich: UV-A 400 - 320 nm, UV-B 320 - 280 nm, UV-C 280 - 200 nm. Geeignete UV-Quats sind beispielsweise in US-A 4061730 und US-A 5427773 beschrieben.

### Besonders geeignete UV-Quats sind

Poly(N-Hydroxypolyoxypropylethyl)-N-(adipylpolyoxypropyl)-N-methyl-N-(2-hydroxy-3-cinnamidopropyl-dimethylammoniumchlorid)-methansulfonate (INCI-Name: Polyquaternium-59 (and) Butylene Glycol), vertrieben von der Firma Croda Inc., Parsippany, NJ 07054, USA, unter dem Handelsnamen Crodasorb® UV-HPP, und

Dodecyl-[3-para-dimethylaminobenzamido)-propyl]-dimethylammonium-toluensulphonat / Propan-1,2-diol-1-octadecanoat (INCI-Name: DimethylPABAmidopropyl Laurdimonium Tosylate (and) Water (and) Propylene Glycol Stearate), vertrieben von der Firma ISP-Chemicals NJ 07470, USA unter dem Handelsnamen Escalol® HP 610.

Im Gegensatz zu UV-Quat, soll unter UV-Filtersubstanz im folgenden eine in kosmetischen Mitteln einsetzbare UV-Licht absorbierende anorganische oder organische Filtersubstanz ohne quaternären Stickstoff verstanden werden.

Als UV-Licht absorbierende anorganische Pigmente kommen alle für dies Verwendung bekannten Pigmente oder Mikropigmente in Betracht, insbesondere in Wasser schwerlösliche oder unlösliche Metallverbindungen oder Halbmetallverbindungen in ionischer als auch nicht-ionischer oder in oxidierter Form. Die Pigmente können in dieser Form einzeln oder als Gemische vorliegen oder einzeln oder als Gemische von Mischoxiden, wobei auch Gemische von Mischoxiden mit Reinoxiden umfasst werden. Als Beispiele hierfür seien genannt Titanoxide (beispielsweise TiO₂), Zinkoxide (beispielsweise ZnO), Aluminiumoxide (beispielsweise Al₂O₃), Eisenoxide (beispielsweise Fe₂O₃), Manganoxide (beispielsweise MnO), Siliciumoxide (beispielsweise SiO₂), Silicate, Ceroxid, Zirkoniumoxide (beispielsweise ZrO₂), Bariumsulfat (BaSO₄) oder Gemische davon. Geeignete Pigmente bzw. Mikropigmente sind kommerziell erhältlich, beispielsweise Hombitec® L5 (INCI Bezeichnung: Titanium Dioxide) von Merck.

Die UV-Licht absorbierenden anorganischen Pigmente können in dem erfindungsgemäßen Mittel in einer Gesamtmenge zwischen 0,01 bis 20,0 Gew.%, insbesondere zwischen 0,05 bis 10,0 Gew.%, vorzugsweise zwischen 0,05 und 5,0 Gew. %, bezogen auf die Menge des Mittels, enthalten sein.

Von den organischen UV-Filtersubstanzen können alle bekannten UVA-, UVBund UVA/UVB-Filtersubstanzen, einzeln oder in Kombination miteinander, verwendet werden, die üblicherweise in kosmetischen und/oder dermatologischen Zubereitungen enthalten sind. Die UV-Filter können öllöslich oder wasserlöslich sein. Da eine bevorzugte Ausführungsform des erfindungsgemäßen Mittels darin besteht, dass die Pigmente in die Wasserphase und die organische UV-Filtersubstanz in die Ölphase eingearbeitet werden, sind für diesen Fall öllösliche UV-Filtersubstanzen bevorzugt.

Für UVA-Filter kommen zum Beispiel die Derivate des Dibenzoylmethans (beispielsweise Parsol 1789 von Givaudan/Roure, INCI-Bezeichung: Butyl Methoxydibenzoylmethane) in Betracht.

Als Beispiele für UVB-Filter können die folgenden Verbindungen genannt werden: Benzylidencampher oder Derivate davon, insbesondere Methylbenzylidencampher (beispielsweise 3-Benzylidencampher, 3-(4-Methylenbenzyliden)-dlcampher), Derivate und Ester der Zimtsäure, insbesondere Derivate und Ester der Methoxyzimtsäure (beispielsweise 4-Methoxyzimtsäureoctylester oder 4-Methoxyzimtsäureisopentylester, 4-Methoxyzimtsäure-2-ethylhexylester (INCI: Ethylhexyl Methoxycinnamate)), Derivate und Ester der Benzoesäure, insbesondere der 4-Aminobenzoesäuren; 2-Cyano-3,3-diphenyl-acrylsäure-2-ethylhexylester (INCI: Octocrylene), Polyhydroxybenzoesäuren (beispielsweise Polyhydroxybenzoesäuremethylester oder Polyhydroxybenzoesäurepropylester), Ester der Salicylsäure (beispielsweise Salicylsäure(2-ethylhexyl)ester oder Salicylsäure(4-isopropylbenzyl)ester).
Als wasserlösliche UV-Filtersubstanzen seien beispielhaft genannt: Sulfonsäuren, Benzophenone und deren Derivate, beispielsweise die Sulfonsäurederivate der Benzophenone (beispielsweise 2-Hydroxy-4-methoxybenzophenon-5-sulfonsäure) als UVB/UVA-Filter oder der Benzimidazole (beispielsweise 2-Phenylbenzimidazol-5-sulfonsäure) sowie deren Salze, insbesondere die Natrium- und Kalium-Salze.

Als weitere UV-Filtersubstanzen kommen in Betracht: Dibenzoylmethane oder geeignete Polypeptide, insbesondere Sauerstoffradikalfänger, beispielsweise die bekannten Mn-, Fe- oder Zn-Superoxiddismutasen, sowie Tocopherole und Vitamine (beispielsweise Ascorbinsäure).

Besonders bevorzugt sind die organischen UV-Filtersubstanzen 4-Methoxyzimtsäure-2-ethylhexylester und 2-Cyano-3,3-diphenyl-acrylsäure-2-ethylhexylester.

Die organischen UVA-, UVB- oder UVB/UVA-Filtersubstanzen können in dem erfindungsgemäßen Mittel in einer Gesamtmenge zwischen 0,1 bis 30,0 Gew.%, insbesondere zwischen 0,5 bis 25,0 Gew.%, vorzugsweise zwischen 0,3 und 5,0 Gew.%, bezogen auf die Menge des Mittels, enthalten sein.

Das Gewichtsverhältnis von UV-Quat zu UV-Filter soll vorzugsweise im Bereich von 1:1 bis 1:15, besonders bevorzugt von 1:1 bis 1:5, ganz besonders bevorzugt von 1:2 bis 1:3, liegen.

Vorteilhafterweise kann das erfindungsgemäße Mittel zusätzlich mindestens ein synthetisches Polymer enthalten. Die synthetischen Polymere sind vorzugsweise Acryl-Polymere, insbesondere aus der Gruppe der Crosspolymere aus Acrylaten und Alkylacrylaten und/oder aus Acrylaten und Allylethern auszuwählen. Beispielsweise eignen sich hierfür Pemulen®-, Carbopol®- und Acrisint®-Typen, beispielsweise Pemulen TR1 von Goodrich, (INCI-Bezeichnung: Acrylates/C10-30 Alkyl Acrylate Crosspolymer), Carbopol 1382 von Goodrich (INCI-Bezeichnung: Acrylates/C10-30 Alkyl Acrylates Crosspolymer), Carbopol 2984 von Goodrich, (INCI-Bezeichnung: Carbomer ) oder Carbopol Ultrez 10 von Goodrich, (INCI-Bezeichnung: Carbomer) oder Acrisint 400 von 3 V (INCI-Bezeichnung: Carbomer), welche einzeln oder in Kombination in dem erfindungsgemäßen Mittel vorliegen können. Die betreffenden synthetischen Polymere können in die Öloder Lipidphase oder in die Wasserphase, bevorzugt in die Wasserphase, des erfindungsgemäßen Mittels eingearbeitet werden und sind darin in einer Gesamtmenge zwischen 0,05 und 5,0 Gew.%, insbesondere zwischen 0,1 und 3,0 Gew.%, ganz besonders zwischen 0,1 und 1,0 Gew.%, bezogen auf die Gesamtmenge des Mittels, enthalten.

Selbstverständlich kann das erfindungsgemäße, zum Abspülen nach der Anwendung bestimmte Mittel alle für derartige Mittel üblichen und bekannten Zusatzstoffe, z. B. Verdickungsmittel, wie beispielsweise, Bentonit, Fettsäuren, Stärke, Polyacrylsäure und deren Derivate, Cellulosederivate, Alginate, Vaseline, Paraffinöle, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester, ferner Trübungsmittel, wie z. B. Polyethylenglykolester, Alkohole, wie z. B. Beispiel Ethanol, Propanol, Isopropanol, Polyole wie zum Beispiel Ethylenglykol, 1,2- oder 1,3-Propandiol, 1,2-, 1,3- oder 1,4-Butandiol, 1,2-, 1,3-, 1,4- oder 1,5-Pentandiol und Glycerin, enthalten. Als geeigneter aromatischer Etheralkohol kommt ein Ethylenglykolether, insbesondere ein Ethylenglykolmonophenylether, in Betracht, beispielsweise Phenoxetol von Nipa (INCI-Bezeichnung: Phenoxyethanol) in Betracht. Weiter geeignete Zusätze sind Zucker wie z. B. D-Glucose, Lösungsvermittler, Stabilisatoren, Puffersubstanzen, Parfümöle, Farbstoffe sowie haarkonditionierende und haarpflegende Bestandteile, wie z. B. kationische Polymere, Lanolinderivate, Cholesterin, Pantothensäure und Betain, enthalten.

Die erwähnten Bestandteile werden in dem Mittel in den für solche Zwecke üblichen Mengen verwendet, z. B. die Netzmittel und Emulgatoren in Konzentrationen von insgesamt 0,2 bis 30 Gew.-%, die Alkohole in einer Menge von 0,5 bis 30 Gew.%, insbesondere von 1 bis 25 Gew.%, vorzugsweise von 2 bis 20 Gew.%, ganz bevorzugt von 3 bis 15 Gew.%, bezogen auf die Gesamtmenge des Mittels; die Trübungsmittel, Parfümöle und Farbstoffe in einer Menge von jeweils 0,01 bis 1 Gew.-%, die Puffersubstanzen in einer Menge von insgesamt 0,1 bis 10 Gew.-%, Zucker, Lösungsvermittler, Stabilisatoren, sowie haarkonditionierende und haarpftegende Bestandteile in einer Menge von jeweils 0,1 bis to Gew.-%, während die Verdickungsmittel und Lösungsvermittler in einer Menge von insgesamt 0,5 bis 20 Gew.-% in diesem Mittel enthalten sein können. Aromatische Etheralkohole können im erfindungsgemäßen Mittel in einer Menge zwischen 0,1 und 10 Gew.%, insbesondere zwischen 0,2 und 5 Gew.%, vorzugsweise zwischen 0,5 und 2 Gew.% vorliegen.

Selbstverständlich kann die erfindungsgemäße Zusammensetzung alle weiteren für Haut- und Haarkosmetika üblichen und bekannten Zusatz-, Hilfs- und Trägerstoffe enthalten.

Grundsätzlich ist dem Fachmann bekannt, welche Zusatz-, Hilfs- und Trägerstoffe in der Haar- und Hautkosmetik verwendet werden, so dass die näheren Ausführungen nur beispielhaften Charakter haben und nur zur weiteren Veranschaulichung der vorliegenden Erfindung dienen sollen.

Im übrigen kann hierzu auf die vorliegende Literatur verwiesen werden, die den allgemeinen Aufbau von Mitteln beschreiben, beispielsweise SCHRADER, K., Grundlagen und Rezepturen der Kosmetika, 2. Auflage, 1989, oder DOMSCH, A., Die kosmetischen Präparate, Verlag für chemische Industrie (H. Ziolkowsky, Ed.), 4. Auflage, 1992, oder LOWE, N. J. & SHAAT, N. A., Suncreens, Development, Evaluation and Regulatory Aspects , Marcel Dekker Inc., 1990.

Als Zusatz-, Hilfs- und Trägerstoffe seien daher beispielhaft nur einige genannt, wobei diese Aufzählung nicht abschließend ist: Verdickungsmittel (wie zum Beispiel Tone, Stärke, Polyacrylsäure und deren Derivate, Zellulosederivate oder Alginate), weitere Haar- und Hautpflegestoffe (wie zum Beispiel, Zucker, Proteine, Lanolinderivate, Vitamine oder Provitamine, beispielsweise Biotin, Vitamin C oder D-Panthenol), Antifettwirkstoffe, anorganische oder organische Säuren (wie zum Beispiel Milchsäure, Citronensäure, Glykolsäure, Phosphorsäure), Konservierungsmittel (wie zum Beispiel Parahydroxybenzoesäure-Ester), nichtwässrige Lösungsmittel, Antioxidantien (wie zum Beispiel Tocopherole oder Ester davon), Farbstoffe sowie Duftstoffe bzw. Parfüme.

Die Zusatz-, Hilfs- und Trägerstoffe können in den für den Fachmann bekannten üblichen Mengen verwendet und nach an sich bekannten Methoden eingearbeitet werden.

Die erfindungsgemäßen Mittel können in verschiedenen Darreichungsformen, wie sie für kosmetische Haut- und Haarprodukte zum Schutz vor Sonnenlicht bekannt sind, vorliegen. Beispielsweise in Form von Shampoos, Rinses, Gelen, Cremegelen, Cremes, Lotionen, Schüttelmixturen, Sprays oder Schäumen.

Bevorzugt ist das zum Ausspülen bestimmte kosmetische Mittel mit Schutzwirkung für Haare und/oder Haut vor UV-Strahlen ein konditionierendes Shampoo, eine konditionierende Rinse oder eine Haarpackung (Rinse-off-Haarkur). Es kann jedoch auch ein Mittel sein, das überwiegend dazu bestimmt ist, das Haar oder die Haut vor UV-Strahlen zu schützen, wie ein Sonnenschutzmittel; auch dieses ist dann jedoch dazu bestimmt, nach dem Auftragen und einwirken lassen, wieder abgespült zu werden. Dennoch bleibt danach die UV-Schutzwirkung erhalten.

Die erfindungsgemäßen Mittel können nach an sich bekannter Weise hergestellt. Das Zusetzen und Vermischen von üblichen Hilfs- und Trägerstoffen kann nach an sich bekannten Methoden erfolgen.

Als organische UV-Filtersubstanz kann natürlich auch eine wasserlösliche UV-Filtersubstanz gewählt werden, die entweder einzeln oder zusammen mit einer öllöslichen UV-Filtersubstanz in die Wasser- bzw. Lipidphase eingearbeitet werden kann.

Demzufolge ist das Verfahren zur Herstellung des erfindungsgemäßen Mittels auch in der Weise möglich, dass die organische UV-Filtersubstanz wasserund/oder öllöslich ist und in die Wasser und/oder Öl- oder Lipidphase eingearbeitet wird.

Vorzugsweise enthält das erfindungsgemäße Mittel jedoch keine öligen bzw. fettigen Substanzen oder nur in einer geringen Menge von 0,1 bis 5 Gew.%, bezogen auf die Gesamtzusammensetzung, und (das damit behandelte Substrat) fühlt sich nicht ölig oder fettig an.

In einem weiteren Verfahren zur Herstellung des erfindungsgemäßen Mittels kann zusätzlich ein synthethisches Polymer hinzugefügt werden, wobei das synthetische Polymer bevorzugt in die Wasserphase eingearbeitet wird. Hierfür werden bevorzugt Acrylpolymere, vorzugsweise ausgewählt aus der Gruppe der Crosspolymere aus Acrylaten und Alkylacrylaten oder aus Acrylaten und Allylethern, verwendet.

Die folgenden Beispiele sollen die vorliegende Erfindung näher erläutern, wobei die angegebenen Prozente Gewichtsprozente darstellen und auf die Gesamtmenge bzw. das Gesamtgewicht der Mittel bezogen sind.

Die in den Beispielen angegebenen Einzelsubstanzen und Rohstoffe sind kommerziell erhältlich; sie können von den nachstehend angegeben Herstellern bezogen werden und tragen folgende INCI Bezeichnungen, wobei auf bekannte Literatur Bezug genommen wird, beispielsweise auf International Cosmetic Ingredient Dictionary and Handbook, Seventh Edition, 1997:

| **Rohstoff/Handelsname** | **INCI-Bezeichnung** | **Hersteller** |
|---|---|---|
| Abil Wax® 9801 D | Cetyl Dimethicone | Goldschmidt |
| Acrisint® 400 | Carbomer | 3 V |
| Amphisol® K | Potassium Cetyl Phosphate | Givaudan/Roure |
| Antaron® V-216 | PVP/Hexadecene Copolymer | ISP |
| Antaron® V-220 | PVP/Eicosene Copolymer | ISP |
| Betain Monohydrat | Betaine | Amino |
| Carbopol® 1382 | Acrylates/C10-30 Alkyl Acrylate Crosspolymer | Goodrich |
| Carbopol® 2984 | Carbomer | Goodrich |
| Carbopol® Ultrez 10 | Carbomer | Goodrich |
| Cetiol® 868 | Octyl Stearate | Cognis |
| Cetiol® SN | Cetearyl Isononanoate | Cognis |
| Colorona® Oriental Beige 17237 | Mica (and) CI 77891 (and) CI 77491 | Merck |
| Crodasorb UV-HPP | Polyquaternium-59 | Croda |
| Dekaben® LMB | lodopropynyl Butylcarbamate | Jan Dekker |
| D-Panthenol | Panthenol | BASF |
| Dragosantol® | Bisabolol | Dragoco |
| Edeta® BD | Disodium EDTA | BASF |
| Empicol ESB 3/WF | Sodium Laureth Sulfate | Huntsman Surface Science |
| Eusolex® 6300 | 4-Methylbenzylidene Camphor | Merck |
| Eutanol® G | Octyldodecanol | Cognis |
| Escalol® HP610 | Dimethyl PABAmidopropyl Laurdimonium Tosylate | Van Dyk |
| Escalol® 507 | Octyl Dimethyl PABA | Van Dyk |
| Extrapon® Hamamelis | Witch Hazel Distillate | Dragoco |
| Finsolv® TN | C12-15 Alkyl Benzoate | Finetex |
| Genamin CTAC 50 | Cetrimonium Chloride | Clariant |
| Hombitec® L5 | Titanium Dioxide | Merck |
| Hydrolite®-5 2/016020 | Pentylene Glycol | Dragoco |
| Lanette® O | Cetearyl Alcohol | Cognis |
| Neo Heliopan® AV/OA | Ethylhexyl Methoxycinnamate | Haarmann & Reimer |
| Neo Heliopan® Typ 303 | Octocrylene | Haarmann & Reimer |
| Parsol® 1789 | Butyl Methoxydibenzoylmethane | Givaudan/Roure |
| Phenoxetol® | Phenoxyethanol | Nipa Laboratories |
| Pemulen® TR 1 | Acrylates/C10-30 Alkyl Acrylate Crosspolymer | Goodrich |
| Salcare SC 92 | Polyquatemium-32 | Ciba |
| Tannogen® LS 1463 C | Hydrolized Soy Protein | Laboratoires Serobiologiques |
| TEGO BETAIN L 5045 | Cocamidopropyl Betaine | Goldschmidt |
| Vitamin E-Acetat | Tocopheryl Acetate | BASF |
| PHB-Methylester | Methylparaben | Chemag |
| PHB-Propylester | Propylparaben | Chemag |
| Ucare Polymer JR 400 | Polyquaternium-10 | Amerchol |

### Beispiel 1 Conditioner mit UV-Filter

| | |
|---|---|
| Lanette O | 3,00 g |
| Genamin CTAC 50 | 1,00 g |
| Neo Heliopan® AV/OA | 2,00 g |
| Neo Heliopan®Typ 303 | 0,50 g |
| Parfum | 0,30 g |
| D-Panthenol | 0,50 g |
| Wasser, vollentsalzt | ad 100,00 g |

### Beispiel 2 Conditioner mit UV-Quat (Stand der Technik)

| | |
|---|---|
| Lanette O | 3,00 g |
| Genamin CTAC 50 | 1,00 g |
| Escalol® HP 610 | 2,00 g |
| Parfum | 0,30 g |
| D-Panthenol | 0,50 g |
| Wasser, vollentsalzt | ad 100,00 g |

### Beispiel 3 Conditioner mit UV-Quat und UV-Filter

| | |
|---|---|
| Lanette O | 3,00 g |
| Genamin CTAC 50 | 1,00 g |
| Escalol® HP 610 | 2,00 g |
| Neo Heliopan® AV/OA | 2,00 g |
| Neo Heliopan® Typ 303 | 0,50 g |
| Parfum | 0,30 g |
| D-Panthenol | 0,50 g |
| Wasser, vollentsalzt | ad 100,00 g |

### Beispiel 4 Shampoo mit UV-Quat und UV-Filter

| | |
|---|---|
| Empicol ESB 3/WF | 30,00 g |
| TEGO BETAIN L 5045 | 7,50 g |
| Ucare Polymer JR 400 | 0,50 g |
| Neo Heliopan® AV/OA | 2,00 g |
| Crodasorb UV-HPP | 1,50 g |
| Parfum | 0,30 g |
| D-Panthenol | 0,50 g |
| Wasser, vollentsalzt | ad 100,00 g |

Im folgenden wird die Schutzwirkung von UV-Quats und UV-Filtem in Mitteln des Standes der Technik mit der synergistischen Wirkung der erfindungsgemäßen Kombination aus UV-Quat und UV-Filter durch verschiedene Untersuchungen bestätigt. Dazu dienen die Messungen der DSC-Peaktemperaturen, wie sie nachfolgend in Tabelle 1 für Rinse-Conditioner dargestellt sind.

**Tabelle 1**

| DSC-Peaktemperaturen verschiedener Rinse-Conditioner | |
|---|---|
| **Muster** | **DSC-Peaktemperatur** [°C] |
| Referenz unbehandelt, unbestrahlt | 143,75 |
| Referenz unbehandelt, bestrahlt | 139,93 |
| Rinse-Conditioner gemäß Beispiel 1mit UV-Filter (Stand der Technik) | 138,80 |
| Rinse-Conditioner gemäß Beispiel 2 mit UV-Quat (Stand der Technik) | 139,83 |
| Rinse-Conditioner gemäß Beispiel 3 mit UV-Quat und UV-Filter (gemäß Erfindung) | 142,95 |

Wie Tabelle 1 zeigt, führt bei Rinse-Off-Conditionern die Kombination von UV-Quats und UV-Filtern zu einer deutlichen Anhebung der DSC-Peaktemperatur, was für eine überraschend deutlich erhöhte Schutzwirkung vor UV-Strahlung spricht, die fast den Wert für unbestrahltes Haar erreicht und damit weit über den Schutz hinausgeht, der sich nur mit UV-Quats oder nur mit UV-Filtern erzielen lässt.

## Patentansprüche

1. Zum Ausspülen bestimmtes kosmetisches Mittel mit Schutzwirkung für Haare und/oder Haut vor UV-Strahlen, **dadurch gekennzeichnet, dass** es eine Kombination aus einem UV-Quat und einer UV-Filtersubstanz enthält.

2. Mittel nach Anspruch 1, **dadurch gekennzeichnet, dass** das UV-Quat ausgewählt ist aus Poly(N-Hydroxypolyoxypropylethyl)-N-(adipylpolyoxypropyl)-N-methyl-N-(2-hydroxy-3-cinnamidopropyldimethylammoniumchlorid)-methansulfonate und Dodecyl-[3-paradimethylaminobenzamido)-propyl]-dimethylammonium-toluensulphonat.

3. Mittel nach einem der Ansprüchen 1 oder 2, **dadurch gekennzeichnet, dass** die UV-Filtersubstanz ausgewählt ist aus der Gruppe der organischen UVA-, UVB- und UVA/UVB-Filtersubstanzen.

4. Mittel nach Anspruch 3, **dadurch gekennzeichnet, dass** die organische UVA-, UVB- und UVA/UVB-Filtersubstanz öllöslich ist.

5. Mittel nach einem der Ansprüchen 1 bis 4, **dadurch gekennzeichnet, dass** der UV-Filter ausgewählt ist aus 4-Methoxyzimtsäure-2-ethylhexylester und 2-Cyano-3,3-diphenyl-acrylsäure-2-ethylhexylester.

6. Mittel nach einem der Ansprüchen 1 bis 5, **dadurch gekennzeichnet, dass** das Gewichtsverhältnis von UV-Quat zu UV-Filter im Bereich von 1:2 bis 1:3 liegt.

7. Mittel nach einem der Ansprüche 1 bis 6, **dadurch gekennzeichnet, dass** es ein Shampoo oder eine Haarspülung (Rinse) ist.

8. Mittel nach einem der Ansprüche 1 bis 7, **dadurch gekennzeichnet, dass** es zusätzlich mindestens ein synthetisches Polymer enthält.

9. Mittel nach Anspruch 8, **dadurch gekennzeichnet, dass** das zusätzliche synthetische Polymer ein Acrylpolymer ist.

10. Mittel nach Anspruch 9, **dadurch gekennzeichnet, dass** das Acrylpolymer ausgewählt ist aus der Gruppe der Crosspolymere aus Acrylaten und Alkylacrylaten oder aus Acrylaten und Allylethern.

11. Mittel nach einem der Ansprüche 1 bis 10, **dadurch gekennzeichnet, dass** es eine haarkonditionierende Rinse oder Haarkurpackung ist.

12. Mittel nach einem der Ansprüche 1 bis 11, **dadurch gekennzeichnet, dass** es keine oder nur eine geringe Menge von 0,1 bis 5 Gew.% öliger Bestandteile enthält und sich nicht ölig anfühlt.

13. Verwendung einer Kombination aus einem UV-Quat und einer UV-Filtersubstanz in einem kosmetischen Mittel mit Schutzwirkung für Haare und/oder Haut vor UV-Strahlen, das dazu bestimmt ist, nach der Anwendung ausgespült bzw. abgespült zu werden.
